# EUROPEAN PATENT APPLICATION

(11) **EP 4 410 097 A1**
(43) Date of publication of application: **07.08.2024**
(21) Application number: 23020061.0
(22) Date of filing: 02.02.2023
(51) Int. Cl.: A01H 1/00, A01H 6/46, C07K 14/415, C12N 15/82

(54) **ERGOT RESISTANCE HAPLOTYPES IN RYE**

(71) Applicant: Nordic Seed A/S, 8464 Galten (DK)
(72) Inventor: Jahoor, Ahmed, 4000 Roskilde (DK); Orabi, Jihad, 8300 Odder (DK); Mahmood, Khalid, 8300 Odder (DK); Sarup, Pernille Merete, 8732 Hovedgaard (DK)
(74) Representative: Andersen, Claus Heide

(57) **Abstract**

The present invention describes a unique haplotype pattern in resistant genotypes that has been shown to increase resistance to noxious ergot (*Clavicep pupurea Fr*) disease in rye (*Secale cereale L*.). The presence of the haplotype is in gene and is responsible for the increased of ergot resistance. Methods for determining the haplotype linked with resistance have also been revealed in this application. Furthermore, the nucleic acid molecule which carries the necessary information as well as a protein which can be encoded by the nucleic acid molecule are also described.

## Description

### Field of invention

The present invention is in the technical field of plant breeding and green biotechnology, specifically in the field of rye breeding employing marker assisted selection and marker technology. For instance, cereals with ergot resistance have been developed using marker assisted selection. They are distinguished by the persistence of unfavorable, typically ergot-causing effects that would otherwise be associated with the introgression of chromosomal segments containing the resistance locus in cultivars. In this respect, the present invention provides plants, in particular rye plants which, as ergot resistant parent, are capable of causing ergot resistance for the Gülzow germplasm, whereby a linkage drag otherwise associated with the ergot resistance property is reduced or even eliminated in rye plants resulting from a cross of these parents having identified haplotype pattern.

In addition, the present invention is concerned with nucleic acid molecules that carry the essential information to generate ergot resistance, as well as a protein that can be coded for by the nucleic acid molecule. Furthermore, the invention concerns the use of the nucleic acid molecules, DNA, and protein in the rye breeding, for example.

### Background

Rye (*Secale cereale* L.) is an important cereal crop, especially in Northern Europe ¹. Rye has a high nutritional value as well as a tolerance to the cool environmental conditions at northern latitudes ¹. Rye can grow in cool temperatures and in marginal soil conditions, which may not be as suitable for wheat and barley¹. However, rye is threatened by ergot, a fungal disease which is known to cause severe problems². Ergot contamination of harvested grains is known to produce serious issues in cross-pollinated rye³. Due to toxic ergot alkaloids (EAs), ergot poses a serious risk to food and feed security. The EAs are a class of secondary metabolites that are defined as 4-( γ,γ,-dimethylallyl) tryptophan derivatives (DMAT)⁴. Their toxicity is linked to their structural similarity to dopamine, noradrenaline, adrenaline, and serotonin, which allows binding to the biogenic amine receptor and the disruption of neurotransmission^{5,6}. Due to food security concerns, ergot is a serious threat to bread production as well feed production for animals. Ingestion of such food or feed containing EAs causes intoxication in humans and animals, as well as disorders like ergotism, which is characterized by symptoms including abdominal discomfort, vomiting, skin burning, sleeplessness, and hallucinations^{7,8}. More than 40 distinct EAs have been identified, with production depending on a variety of criteria including sclerotia development, fungal strain, host cereal, geographical region, and climate conditions. The ergometrine, ergotamine, ergosine, ergocristine, ergokryptine, and ergocornine are the most common EAs that are already listed in European guidelines as regards maximum levels of ergot sclerotia and ergot alkaloids in certain foodstuffs⁹. There are strict European Union regulations on the use of rye for human consumption due to the persistent presence of hazardous ergot alkaloids¹⁰. As a result, until 2024, the milling industry in the European Union will only accept trace amounts of sclerotia in harvested grains for bread manufacturing processes³. The sclerotia are compact masses of hardened fungal mycelia and important for fungal survival during adverse environmental conditions. From July 2024, the sclerotia threshold in harvested grain for final consumers will be reduced even further to 250 µg/kg¹¹. In this context, the six most common EAs (mentioned above) with their inine epimers are to be restricted in unprocessed rye from that date to a maximum total content of 250 µg/kg. Rye farmers may suffer significant financial losses if ergot contamination levels exceed the EU regulatory threshold. The European directive on undesirable substances in animal feed fixes a limit of 0.1% (1g/kg) for ergot sclerotia in feedstuff containing unground cereals. For a reliable marketing of rye products, compliance with these ergot contamination standards in the harvest is essential. Ergot infection is thus one of the most economically important diseases in rye, and it must be avoided in commercial production.

The *C. purpurea* infects ovaries in plants, with no obvious signs of plant response. Furthermore, any quantitative trait loci (QTL), haplotype or gene responsible for causing resistance in cereals had not been identified until recently. It is critical to identify an ergot resistance and the cause of this resistance and later use this in breeding to develop rye plants that are resistant to ergot infections for food security.

### Methodology

### Semi-field trial and ergot resistance phenotyping

The phenotyping for ergot resistance was carried out on the panel of 190 genotypes in semi-field environment. Eight seeds from 190 inbred genotypes were planted in 104-hole trays filled with fine-grain peat soil. Plants were then transferred to the greenhouse facilities at Nordic Seed A/S and cultivated in 16 hours of daylight at 18-24°C and 8 hours of dark at 14-16°C for germination. After two weeks, plants sown in trays were placed in a cold room for vernalization for 8 weeks. The conditions were maintained at 4 °C, 8/16 h day/night cycle, 120 µE m⁻² s⁻¹ light intensity and 80% humidity. Plants were transferred into pots (diameter 15 cm) after vernalization, with four plants in each pot and two replications for each genotype. The vernalized plant seedlings that had been sown in pots were transplanted into semi-field conditions. At flowering initiation stage, spikes were inoculated with freshly prepared spore suspension of C. *purpurea.* Artificial inoculation was performed twice: once at 50% flowering and again at 100% flowering. Inoculum was produced on wheat grains as described by Engelke et al., (2002) with slight modifications¹². Briefly, fresh *C. purpurea* culture were propagated on potato dextrose agar (PDA, Difco) medium at room temperature. C. *purpurea* conidia spores were produced on autoclaved wheat grains. The inoculated wheat kernels were incubated at 17-19 °C for 21 days. Conidial suspensions from *C*. *purpurea* growth were harvested in sterile water, filtered through cheesecloth, and concentrations determined with a hemocytometer by microscopy. Spore concentration at 1×10⁶ conidia/ml was used for inoculation.

The rye inbred lines were phenotyped for their resistance response towards *C*. *purpurea.* We measured the number of infected and non-infected spikes and calculated % infection. We had also measured the number of ergot sclerotia per plant and calculated % number of ergots per 100 spikes. The percent weight of ergot sclerotia was determined through weighing the sclerotia in the total grain weight of specific rye genotypes harvested from each pot.

### Material, DNA extraction and genotyping

*A panel of 190 inbred rye (Secale cereale L.) lines comprising of 101 restorer and 89 non-restorer germplasm were selected. Two seeds from each genotype were planted in 104-hole trays filled with fine-grain peat soil. Plants were then transferred to the greenhouse facilities at Nordic Seed A*/*S and cultivated in 16 hours of daylight at 18-24°C and 8 hours of dark at 14-16°C for germination. After seven days, the lowest section of two coleoptiles and primary leaves were cut, equivalent to 75 mg plant material, and placed in a 96-well Micro-Dilution Tube System (STARLAB International GmbH) containing two 4 mm glass beads per 1.2 mL tubes. Plant tissue samples were stored at -20°C for two days prior to freeze drying for an additional two days. DNA extraction was done using an adapted SDS-based method according to Pallotta, et al. ¹³. DNA concentration and 260*/*280nm ratio of samples were measured using Epoch^{™} microplate spectrophotometer (Biotek^{®}) and evidence of fragmentation by size-visualization on 1.2% agarose gel. Samples of each line containing 200 ng high molecular weight gDNA with ≥ 1.8 260*/*280nm ratio were genotyped using a custom Illumina Infinium 15Kwheat and 5K Rye single nucleotide polymorphism (SNR) array, denoted as 20K. The mapping position of SNR markers was found by mapping the marker sequences to the 'Lo7' reference genome ¹⁴using the NCBI blastn (v. 2.9.0+, ML, USA) function at a significance threshold of the e-value at 10⁻⁵, selecting the physical position of the top hit. Genetic analysis of SNR marker data was performed in R studio (v. 1.1.463, Boston, MA, USA) interface in R statistical software (v. 3.6.3). Prior to analysis, markers were filtered for marker allele frequency ≥0.005, missing individual score ≤ 0.2 and missing marker score ≤ 0.1 to identify informative markers.*

### Genome-wide association study

Discovery of ergot resistance associated SNP markers was done by genome-wide association study (GWAS) using genomic association and prediction integration tool (GAPIT) (v.3) package in R ¹⁵. Manhattan plot was colorized using RColorBrewer (v.1.1-2) R package color palette ¹⁶. GWAS using mixed linear model (MLM) done to identify discrete haplotype blocks associated with ergot resistance. A standard Bonferroni-corrected threshold of α = 0.05 was used as the significance threshold.

### Association genetics and resistance gene enrichment sequencing analysis

The discovery of contigs harboring causal resistance (R) genes has been made possible using a combination of Resistance Gene Enrichment Sequencing (RenSeq) and k-mer association genetics (AgRenSeq). Twenty genotypes were chosen for single-molecule real-time AgRenSeq analysis based on ergot phenotyping. The cerealspecific nucleotide-binding leucine-rich repeat (NLR) bait library (Tv_1) designed by Steuernagel, et al. ¹⁷ was utilized to capture resistance genes in rye (Nikolaj et al, submitted). The bait library contains sixty thousand baits, each of which is 120 nucleotides long. The baits were created utilizing the cereals' conserved motif domains of NLR. The bait library is available at (https://github.com/steuernb/MutantHunter/). Arbor Biosciences was hired to sequence the 20 lines (Michigan, USA). Each line received a minimum of 5g DNA, displaying a UV spectrometry 260:280 ratio of 1.8-2.0 and a high molecular weight modal length of 10 kbp. On a Pacbio^{®} Sequell II long read SMRT device, target enrichment was sequenced, yielding 1.5 Gb of high-quality circular consensus sequences (CCS) data per line. The circular consensus sequences (CCS) were assembled with HiCanu (v. 2.0; -pacbio-hifi, trimReadsCoverage = 2, errorRate = 0.01, genomeSize = 8.8m, minOverlapLength = 500, minReadLength = 1000). By pooling k-mers from all twenty genotypes in a binary format with 1 'present') and 0 ('absent,' a collective K-mer absence/presence matrix was created. The matrix was filtered for rare k-mers present in contigs ≤ 3 lines. K-mer processing of the reads was done following Java source coding published at https://github.com/steuernb/ AgRenSeq.

Identification of candidate ergot resistance genes amongst identified contigs was done following the Java pipeline provided by Arora, et al. ¹⁸ at https://github.com/steuernb/AgRenSeq.

For the purpose of further characterization of the identified contig discovered by AgRenSeq, a manual reference assembly was conducted. The NCBI blastn (v. 2.9.0+) program was used to identify mapping position of the identified contig in the line in which it was detected¹⁹. The AUGUSTUS (3.4.0) algorithm was used to estimate gene structure and extract protein sequence from manually constructed consensus NLR contigs ²⁰. InterPro Scan was utilized for functional analysis and protein domain structure prediction ²¹.

### Sanger sequencing, development and validation of KASP markers

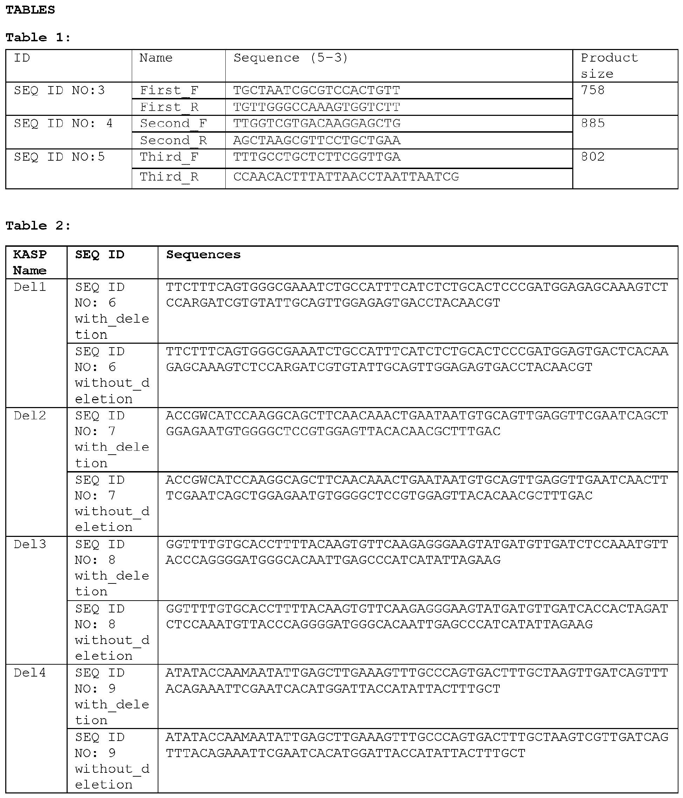

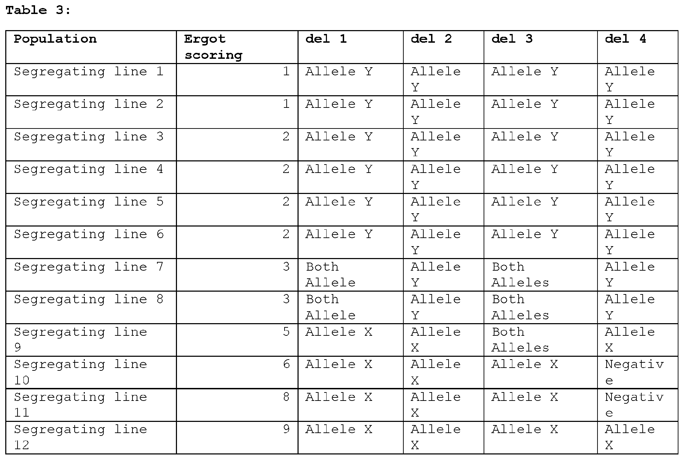

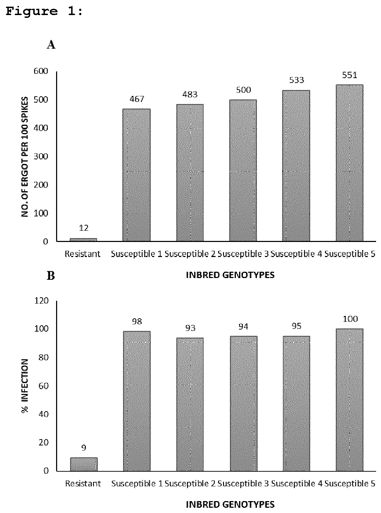

A panel of six selected inbred genotypes of rye based on ergot phenotyping was investigated for the selected region in the identified contig. DNA extraction was done using an adapted SDS-based method with modification as described by Pallotta, et al. ¹³. DNA concentration and 260/280nm ratio of samples were measured using Epoch^{™} microplate spectrophotometer (Biotek^{®}) and DNA integrity through size-visualization on 1.2% agarose gel. The DNA of selected resistant and susceptible genotypes were used in PCR to amplify a predicted gene fragment with overlapping primer pairs shown in Table 1. First of all, sanger sequencing of the identified gene carried out on the five most susceptible genotypes and one highly ergot resistant genotype. The resistance and susceptibility level of selected inbred genotypes has been demonstrated in Figure 1. PCR products were analyzed on 1% agarose gels to visualize the fragments of expected size before dispatch to Macrogen (Netherlands) for sanger sequencing. Identification of single nucleotide variants (SNV) and insertions/deletions (indels) between candidate gene alleles was done by multiple sequence alignment using Multiple Sequence Comparison by Log-Expectation (MUSCLE) method in Geneious Prime (v. 2020.2.3) with ≤ 10 iterations. The identified indels were used for designing a competitive allele-specific PCR (KASP) assay. Four KASP markers were designed to detect the four deletions i.e. dell, del2, del3 and del4. The KASP markers designed by LGC genomics (United Kingdom, UK). The in-house validation of these KASPs were assessed on selected 190 inbred genotypes with low and high ergot resistance.

For the validation, twelve independent progenies (F5) derived from the resistant genotype were sown in field conditions. These plants represent the segregating population and has been screened for the resistance towards ergot in the field conditions. Trials were sown in a Seedmatic^{®} layout with a single parcel (1.0m × 1.25m) consisting of six rows of approximately 25 plants per progeny with a between-row distance of 25 cm and between-parcel distance of 40 cm. Field trial was established in three replications and scored for ergot using a 1-9 scoring scale with 1 as no spike with ergot infection and 9 as all spikes infected with ergot. The progenies were scored by evaluating all plants of the individual progeny within the plot. The plants representing all 12 progenies were also screened with these KASP markers for validation.

### Results

### Genome-Wide Association Study

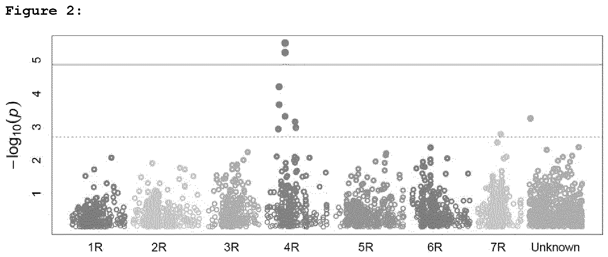

The number of ergots sclerotia per plant was used as phenotypic input in a genome-wide association study (GWAS) to find the ergot resistant chromosomal region in the germplasm. As demonstrated in the Manhattan plot, the 20 K GWAS analysis yielded two significant SNP markers (Figure 2). The highest related marker is "AX 99567860," which is located at 635.9 Mbp on 4R rye chromosome. One NB-LRR is situated at 613 Mbp in the Lo7 reference genome, whereas the other is positioned at 657 Mbp.

The 0.78 (h²) heritability was estimated by dividing the additive genetic variance (total variance of GEBVs) with total phenotypic variance. These results revealed that variance in resistance to ergot in rye is highly due to additive genetic variation.

### Association genetics and resistance gene enrichment sequencing analysis

Using a combination of resistance gene enrichment sequencing (RenSeq) and k-mer association genetics, contigs harboring the causal resistance (R) gene were found. This investigation led to the discovery of a contig in the ergot resistant genotype. This contig is abbreviated as "Tig00000140," presented as SEQ ID NO 10 and it has 12812 nucleotide bases. This resistance-associated contig has partial alignment on the Lo7 reference genome, where only the last 5426 bp aligned on the 4R. On 4R, the alignment position is 651.8 Mbp. According to the NLR annotator, the NLR gene in this contig is located between 4057 and 8382 bp, and this segment did not align to any chromosome of the Lo7 reference genome. However, this specific fragment of contig is present only in our resistant genotypes.

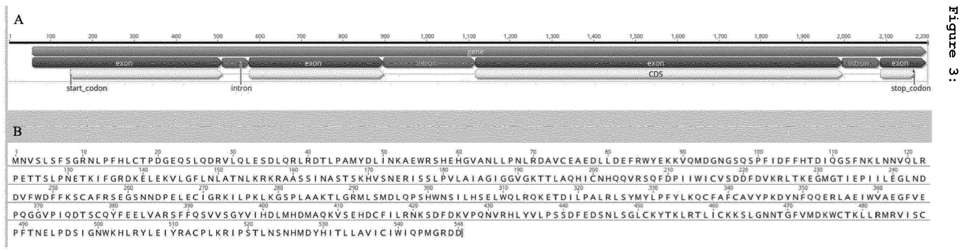

This leads to the conclusion that NB-LRR gene in this contig is not present in the reference Lo7 genome. The AUGUSTUS predicted a complete gene of 2200 nucleotide bases with four exon and three introns (Figure 3A). The coding sequence is translated into 548 amino acids with conserved domains such as Rx_N terminal domain/CC domain (27-91 aa), NB-ARC domain (178-306) and leucine rich repeat (LRR) superfamily. There are five potential LRR motifs predicted in this gene (426-441, 451-466, 478-493, 499-514 and 520-536 aa). This gene is also predicted to contain the functional domain of a P-loop containing triphosphate (120-341 aa), apoptotic protease activating factors (241-324 aa) and winged helix-like binding domain (325-410 aa). All these domains are shown in figures 3A and 3B.

### Sanger sequencing

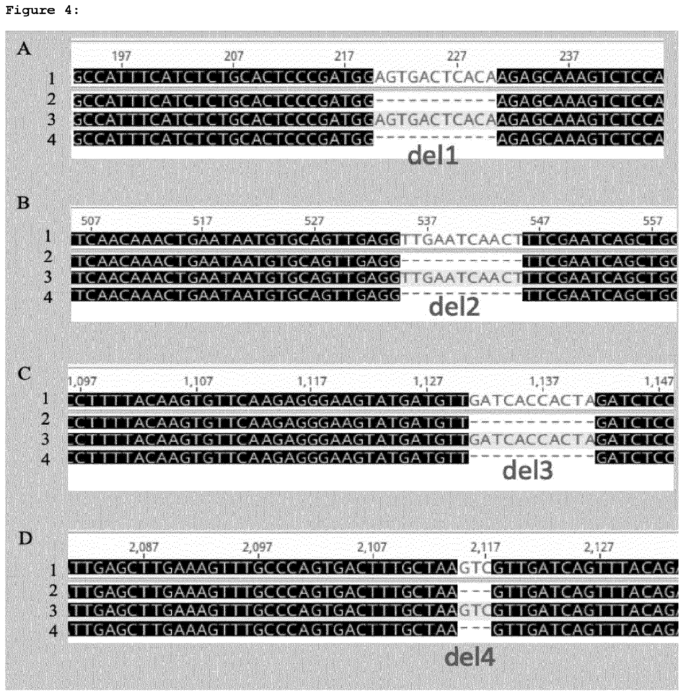

Sanger sequencing of selected six inbred genotypes has been carried out using the specific primers of the NB-LRR gene in the identified contig. In addition, the RenSeq study provided us with the nucleotide sequences of ergot susceptible and resistant genotypes. Nucleotide sequences for twenty inbred genotypes were obtained from a RenSeq investigation. The existence of four deletions in the ergot resistant genotypes was discovered after aligning the consensus sequences from susceptible genotypes to the consensus sequences of resistant genotypes. Among these four deletions, deletion 1 (dell) is located in the coding region (Figure 4). This is 11 bp deletion located in the CC domain. the remaining three deletions (del2, del3 and del4) were found on different introns (Figure 4). The del2 and del3 are also 11 nucleotide bp deletions, whereas del4 is 3 bp nucleotide deletion (Figure 4). The ergot resistant genotypes always exhibit this specific haplotype pattern of four deletions.

### KASP markers development and screening of the inbred elite breeding germplasm

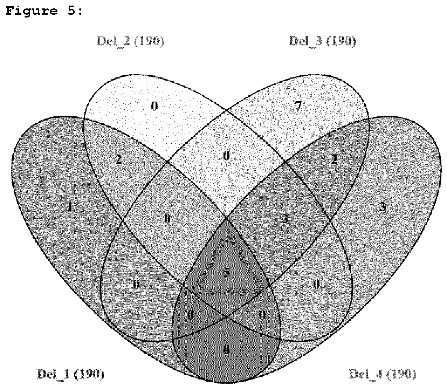

KASP markers were used to screen elite Nordic seed germplasm. Four KASP markers were designed to detect the four deletions, i.e. dell, del2, del3 and del4 (Table 2). The KASP assay demonstrated that it discriminates between the 190 inbred lines examined based on their response to C. purpurea. Eight inbred genotypes with dell were detected by KASP primer flanking dell, whereas KASP with del2 found ten inbred genotypes with this specific deletion (Figure 5). Similarly, KASP with del3 identified 17 inbred genotypes, while KASP with del4 identified 13. Out of 190 genotypes analyzed, KASP data confirmed the presence of all four deletions in five resistant inbred genotypes (Figure 5).

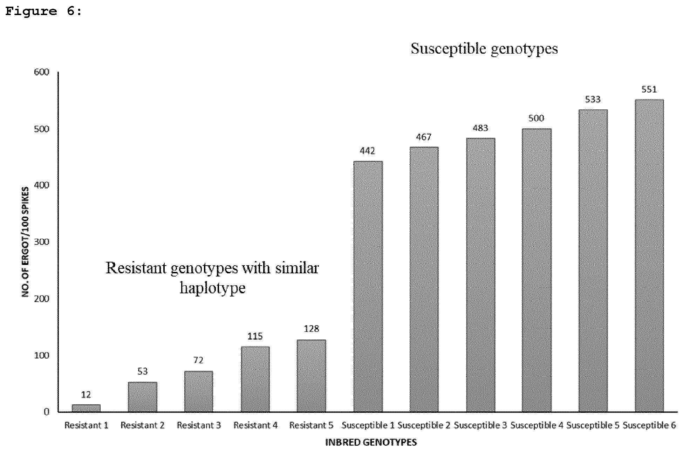

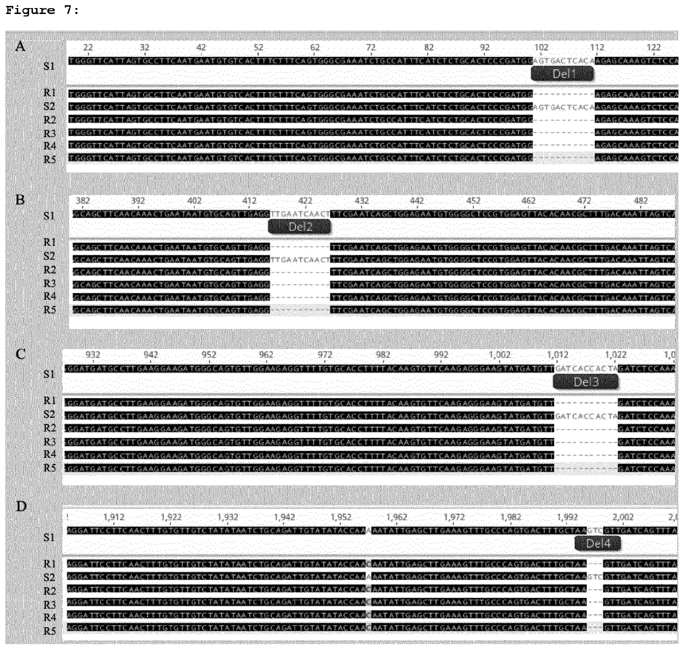

We investigated the response of these five genotypes towards ergot and discovered that the resistance level was very high in these genotypes compared to susceptible genotypes (Figure 6). This supports the assumption that this specific haplotype pattern is responsible for ergot resistance. We amplified the targeted region and sent it for sequencing to validate the presence of these four deletions in these five ergot resistant genotypes. The existence of all four deletions in all five ergot resistance genotypes was confirmed by sanger sequencing (Figure 7). The lines (F5) derived from the ergot resistant genotype have been tested with all four KASP markers. Plants were used to represent the segregating population and have been screened for the resistance towards ergot in the field conditions. In the progeny, scoring of these individuals identify four susceptible plants, two intermediate and six highly resistant progenies derived from segregating lines of resistant genotype (Table 3).

### References

1 Geiger, H. & Miedaner, T. in Cereals 157-181 (Springer, 2009).
2 Lee, M. The history of ergot of rye (Claviceps purpurea) I: from antiquity to 1900. The journal of the Royal College of Physicians of Edinburgh 39, 179-184 (2009).
3 Tudzynski, P., Correia, T. & Keller, U. Biotechnology and genetics of ergot alkaloids. Applied microbiology and biotechnology 57, 593-605 (2001).
4 Florea, S., Panaccione, D. G. & Schardl, C. L. Ergot alkaloids of the family Clavicipitaceae. Phytopathology 107, 504-518 (2017).
5 Klotz, J. L. Activities and effects of ergot alkaloids on livestock physiology and production. Toxins 7, 2801-2821 (2015).
6 Mulac, D. & Humpf, H.-U. Cytotoxicity and accumulation of ergot alkaloids in human primary cells. Toxicology 282, 112-121 (2011).
7 Komarova, E. & Tolkachev, O. The chemistry of peptide ergot alkaloids. Part 1. Classification and chemistry of ergot peptides. Pharmaceutical Chemistry Journal 35, 504-513 (2001).
8 Van Dongen, P. W. & de Groot, A. N. History of ergot alkaloids from ergotism to ergometrine. European Journal of Obstetrics & Gynecology and Reproductive Biology 60, 109-116 (1995).
9 Chain, E. P. o. C. i. t. F. Scientific Opinion on Ergot alkaloids in food and feed. EFSA Journal 10, 2798 (2012).
10 Streit, E. et al. Current situation of mycotoxin contamination and cooccurrence in animal feed-Focus on Europe. Toxins 4, 788-809 (2012).
11 (EU), C. R. Commission Regulation (EU) 2021/1408 of 27 August 2021 amending Regulation (EC) No 1881/2006 as regards maximum levels of tropane alkaloids in certain foodstuffs *Regulation (EC) No 1881*/*2006* (2021).
12 Engelke, T. Ansätze für eine integrierte Bekämpfung des Mutterkorns (Claviceps purpurea [Fr.] Tul.) im Roggen. (Cuvillier Verlag, 2002).
13 Pallotta, M. A. et al. Marker assisted wheat breeding in the southern region of Australia. Proceedings of the Tenth International Wheat Genetics Symposium, 789-791 (2003).
14 Rabanus-Wallace, M. T. et al. Chromosome-scale genome assembly provides insights into rye biology, evolution and agronomic potential. Nature genetics 53, 564-573 (2021).
15 Lipka, A. E. et al. GAPIT: genome association and prediction integrated tool. Bioinformatics 28, 2397-2399, doi:10.1093/bioinformatics/bts444 (2012).
16 Wickham, H. ggplot2. Wiley Interdisciplinary Reviews: Computational Statistics 3, 180-185, doi:10.1002/wics.147 (2011).
17 Steuernagel, B. et al. Rapid cloning of disease-resistance genes in plants using mutagenesis and sequence capture. Nature Biotechnology 34, 652-655, doi:10.1038/nbt.3543 (2016).
18 Arora, S. et al. Resistance gene cloning from a wild crop relative by sequence capture and association genetics. Nature Biotechnology 37, 139-143, doi:10.1038/s41587-018-0007-9 (2019).
19 NCBI. *National Center for Biotechnology Information,* <https://www.ncbi.nlm.nih.gov> (2021).
20 Stanke, M., Steinkamp, R., Waack, S. & Morgenstern, B. AUGUSTUS: a web server for gene finding in eukaryotes. Nucleic Acids Research 32, W309-312, doi:10.1093/nar/gkh379 (2004).
21 Jones, P. et al. InterProScan 5: genome-scale protein function classification. Bioinformatics 30, 1236-1240 (2014).

This XML file does not appear to have any style information associated with it. The document tree is shown below.

<ST26SequenceListing dtdVersion="V1_3" fileName="Ergot patent.xml" softwareName="WIPO Sequence" softwareVersion="2.2.0" productionDate="2023-02-02">
<ApplicationIdentification>
<IPOfficeCode>EP</IPOfficeCode>
<ApplicationNumberText/>
<FilingDate/>
</ApplicationIdentification>
<ApplicantFileReference>Ergot_patent</ApplicantFileReference>
<EarliestPriorityApplicationIdentification>
<IPOfficeCode>DK</IPOfficeCode>
<ApplicationNumberText>PA 2022 00100</ApplicationNumberText>
<FilingDate>2022-02-04</FilingDate>
</EarliestPriorityApplicationIdentification>
<ApplicantName languageCode="da">Nordid Seed A/S</ApplicantName>
<InventionTitle languageCode="en">Rye Resistance Haplotype</InventionTitle>
<SequenceTotalQuantity>18</SequenceTotalQuantity>
<SequenceData sequenceIDNumber="1">
<INSDSeq>
<INSDSeq_length>2200</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..2200</INSDFeature_location>
<INSDFeature_quals>
<INSDQualifier>
<INSDQualifier_name>mol_type</INSDQualifier_name>
<INSDQualifier_value>genomic DNA</INSDQualifier_value>
</INSDQualifier>
<INSDQualifier id="q19">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>attactgcaatctctgacttggtgtatagaaatttttacttcctgtgctaatcgcgtccactgttggcctgcagaacgaaaataggcgtccagactctaacaaccaaccatgagctcatctgccattgg
</INSDSeq>
</SequenceData>
<SequenceData sequenceIDNumber="2">
<INSDSeq>
<INSDSeq_length>2235</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..2235</INSDFeature_location>
<INSDFeature_quals>
<INSDQualifier>
<INSDQualifier_name>mol_type</INSDQualifier_name>
<INSDQualifier_value>genomic DNA</INSDQualifier_value>
</INSDQualifier>
<INSDQualifier id="q20">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>attactgcaatctctgacttggtgtataatttttttttacttcctgtgctaatcgcgtccactgttggcctgcagaacgaaaatagacgtccagactctaacaaccaaccatgagctcatctgccattgg
</INSDSeq>
</SequenceData>
<SequenceData sequenceIDNumber="3">
<INSDSeq>
<INSDSeq_length>20</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..20</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q21">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>tgctaatcgcgtccactgtt</INSDSeq_sequence>
</INSDSeq>
</SequenceData>
<SequenceData sequenceIDNumber="4">
<INSDSeq>
<INSDSeq_length>20</INSDSeq_length>
<INSDSeq_moltype>DNA</INSDSeq_moltype>
<INSDSeq_division>PAT</INSDSeq_division>
<INSDSeq_feature-table>
<INSDFeature>

<INSDFeature_key>source</INSDFeature_key>
<INSDFeature_location>1..20</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q22">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>tgttgggccaaagtggtctt</INSDSeq_sequence>
</INSDSeq>
</SequenceData>
<SequenceData sequenceIDNumber="5">
<INSDSeq>
<INSDSeq_length>20</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..20</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q23">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>ttggtcgtgacaaggagctg</INSDSeq_sequence>
</INSDSeq>
</SequenceData>
<SequenceData sequenceIDNumber="6">
<INSDSeq>
<INSDSeq_length>20</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..20</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q24">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>agctaagcgttcctgctgaa</INSDSeq_sequence>
</INSDSeq>
</SequenceData>
<SequenceData sequenceIDNumber="7">
<INSDSeq>
<INSDSeq_length>20</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..20</INSDFeature_location>
<INSDFeature_quals>
<INSDQualifier>
<INSDQualifier_name>mol_type</INSDQualifier_name>
<INSDQualifier_value>other DNA</INSDQualifier_value>
</INSDQualifier>
<INSDQualifier id="q25">
<INSDQualifier_name>organism</INSDQualifier_name>
<INSDQualifier_value>Secale cereale L</INSDQualifier_value>
</INSDQualifier>
</INSDFeature_quals>
</INSDFeature>
</INSDSeq_feature-table>
<INSDSeq_sequence>tttgcctgctcttcggttga</INSDSeq_sequence>
</INSDSeq>
</SequenceData>
<SequenceData sequenceIDNumber="8">
<INSDSeq>
<INSDSeq_length>27</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..27</INSDFeature_location>

        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q26">
            <INSDQualifier_name>organism</INSDQualifier_name>
            <INSDQualifier_value>Secale cereale L.</INSDQualifier_value>
          </INSDQualifier>
        </INSDFeature_quals>
      </INSDFeature>
    </INSDSeq_feature-table>
    <INSDSeq_sequence>ccaacactttattaacctaattaatcg</INSDSeq_sequence>
  </INSDSeq>
</SequenceData>
<SequenceData sequenceIDNumber="9">
  <INSDSeq>
    <INSDSeq_length>100</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..100</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q27">
            <INSDQualifier_name>organism</INSDQualifier_name>
            <INSDQualifier_value>Secale cereale L.</INSDQualifier_value>
          </INSDQualifier>
        </INSDFeature_quals>
      </INSDFeature>
    </INSDSeq_feature-table>
    <INSDSeq_sequence>ttctttcagtgggcgaaatctgccatttcatctctgcactcccgatggagagcaaagtctccargatcgtgtattgcagttggagagtgacctacaacgt</INSDSeq_sequence>
  </INSDSeq>
</SequenceData>
<SequenceData sequenceIDNumber="10">
  <INSDSeq>
    <INSDSeq_length>111</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..111</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q28">
            <INSDQualifier_name>organism</INSDQualifier_name>
            <INSDQualifier_value>Secale cereal L.</INSDQualifier_value>
          </INSDQualifier>
        </INSDFeature_quals>
      </INSDFeature>
    </INSDSeq_feature-table>
    <INSDSeq_sequence>ttctttcagtgggcgaaatctgccatttcatctctgcactcccgatggagtgactcacaagagcaaagtctccargatcgtgtattgcagttggagagtgacctacaacgt</INSDSeq_sequence
  </INSDSeq>
</SequenceData>
<SequenceData sequenceIDNumber="11">
  <INSDSeq>
    <INSDSeq_length>100</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..100</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q29">
            <INSDQualifier_name>organism</INSDQualifier_name>
            <INSDQualifier_value>Secale cereale L.</INSDQualifier_value>
          </INSDQualifier>
        </INSDFeature_quals>
      </INSDFeature>
    </INSDSeq_feature-table>
    <INSDSeq_sequence>accgwcatccaaggcagcttcaacaaactgaataatgtgcagttgaggttcgaatcagctggagaatgtggggctccgtggagttacacaacgctttgac</INSDSeq_sequence>
  </INSDSeq>
</SequenceData>
<SequenceData sequenceIDNumber="12">
  <INSDSeq>
    <INSDSeq_length>111</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..111</INSDFeature_location>
        <INSDFeature_quals>
          <INSDQualifier>

<INSDQualifier_name>mol_type</INSDQualifier_name>
          <INSDQualifier_value>other DNA</INSDQualifier_value>
        </INSDQualifier>
        <INSDQualifier id="q30">
          <INSDQualifier_name>organism</INSDQualifier_name>
          <INSDQualifier_value>Secale cereale L.</INSDQualifier_value>
        </INSDQualifier>
      </INSDFeature_quals>
    </INSDFeature>
  </INSDSeq_feature-table>
  <INSDSeq_sequence>accgwcatccaaggcagcttcaacaaactgaataatgtgcagttgaggttgaatcaactttcgaatcagctggagaatgtggggctccgtggagttacacaacgctttgac</INSDSeq_sequence
</INSDSeq>
</SequenceData>
<SequenceData sequenceIDNumber="13">
  <INSDSeq>
    <INSDSeq_length>100</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..100</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q31">
            <INSDQualifier_name>organism</INSDQualifier_name>
            <INSDQualifier_value>Secale cereale L.</INSDQualifier_value>
          </INSDQualifier>
        </INSDFeature_quals>
      </INSDFeature>
    </INSDSeq_feature-table>
    <INSDSeq_sequence>ggttttgtgcaccttttacaagtgttcaagagggaagtatgatgttgatctccaaatgttacccaggggatgggcacaattgagcccatcatattagaag</INSDSeq_sequence>
  </INSDSeq>
</SequenceData>
<SequenceData sequenceIDNumber="14">
  <INSDSeq>
    <INSDSeq_length>111</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..111</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q32">
            <INSDQualifier_name>organism</INSDQualifier_name>
            <INSDQualifier_value>Secale cereale L.</INSDQualifier_value>
          </INSDQualifier>
        </INSDFeature_quals>
      </INSDFeature>
    </INSDSeq_feature-table>
    <INSDSeq_sequence>ggttttgtgcaccttttacaagtgttcaagagggaagtatgatgttgatcaccactagatctccaaatgttacccaggggatgggcacaattgagcccatcatattagaag</INSDSeq_sequence
  </INSDSeq>
</SequenceData>
<SequenceData sequenceIDNumber="15">
  <INSDSeq>
    <INSDSeq_length>100</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..100</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>
          </INSDQualifier>
          <INSDQualifier id="q33">
            <INSDQualifier_name>organism</INSDQualifier_name>
            <INSDQualifier_value>Secale cereale L.</INSDQualifier_value>
          </INSDQualifier>
        </INSDFeature_quals>
      </INSDFeature>
    </INSDSeq_feature-table>
    <INSDSeq_sequence>atataccaamaatattgagcttgaaagtttgcccagtgactttgctaagttgatcagtttacagaaattcgaatcacatggattaccatattactttgct</INSDSeq_sequence>
  </INSDSeq>
</SequenceData>
<SequenceData sequenceIDNumber="16">
  <INSDSeq>
    <INSDSeq_length>103</INSDSeq_length>
    <INSDSeq_moltype>DNA</INSDSeq_moltype>
    <INSDSeq_division>PAT</INSDSeq_division>
    <INSDSeq_feature-table>
      <INSDFeature>
        <INSDFeature_key>source</INSDFeature_key>
        <INSDFeature_location>1..103</INSDFeature_location>
        <INSDFeature_quals>
          <INSDQualifier>
            <INSDQualifier_name>mol_type</INSDQualifier_name>
            <INSDQualifier_value>other DNA</INSDQualifier_value>

</INSDQualifier>
<INSDQualifier id="q34">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>atataccaamaatattgagcttgaaagtttgcccagtgactttgctaagtcgttgatcagtttacagaaattcgaatcacatggattaccatattactttgct</INSDSeq_sequence>
</INSDSeq>
</SequenceData>
<SequenceData sequenceIDNumber="17">
<INSDSeq>
<INSDSeq_length>12812</INSDSeq_length>
<INSDSeq_moltype>DNA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..12812</INSDFeature_location>
<INSDFeature_quals>
<INSDQualifier>
<INSDQualifier_name>mol_type</INSDQualifier_name>
<INSDQualifier_value>genomic DNA</INSDQualifier_value>
</INSDQualifier>
<INSDQualifier id="q35">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>ggtagtcttatttgtactctgtagtgaacatgtttttgctagctgaactttacaaagagcttagcctcgtcatccatcgtgcactggtctctctctctccacctcatccctgcctttgatctctaggtg
</INSDSeq>
</SequenceData>
<SequenceData sequenceIDNumber="18">
<INSDSeq>
<INSDSeq_length>548</INSDSeq_length>
<INSDSeq_moltype>AA</INSDSeq_moltype>
<INSDSeq_division>PAT</INSDSeq_division>
<INSDSeq_feature-table>
<INSDFeature>
<INSDFeature_key>source</INSDFeature_key>
<INSDFeature_location>1..548</INSDFeature_location>
<INSDFeature_quals>
<INSDQualifier>
<INSDQualifier_name>mol_type</INSDQualifier_name>
<INSDQualifier_value>protein</INSDQualifier_value>
</INSDQualifier>
<INSDQualifier id="q18">
<INSDQualifier_name>organism</INSDQualifier_name>
<INSDQualifier_value>Secale cereale L.</INSDQualifier_value>
</INSDQualifier>
</INSDFeature_quals>
</INSDFeature>
</INSDSeq_feature-table>
<INSDSeq_sequence>MNVSLSFSGRNLPFHLCTPDGEQSLQDRVLQLESDLQRLRDTLPAMYDLINKAEWRSHEHGVANLLPNLRDAVCEAEDLLDEFRWYEKKVQMDGNGSQSPFIDFFHTDIQGSFNKLNNVQLRPETTSLP
</INSDSeq>
</SequenceData>
</ST26SequenceListing>

## Claims

1. An oligonucleotide which has one of the following nucleotide sequences:
i. SEQ ID NO: 3 First_F and First_R
ii. SEQ ID NO: 4 Second_F and Second_R
iii. SEQ ID NO: 5 Third_F and Third_R
iv. SEQ ID NO: 6 with_deletion
v. SEQ ID NO: 7 with_deletion
vi. SEQ ID NO: 8 with_deletion
vii. SEQ ID NO: 9 with_deletion

2. A nucleic acid molecule which has nucleotide sequence selected from the group consisted of:
i. Nucleotide sequences consisting of SEQ ID NO:1 Resistant
ii. Nucleotide sequences consisting of codes for an amino acid sequence with one of SEQ ID NO: 11
iii. A nucleotide sequence which has an identity of at least 98% with nucleotide sequence in accordance with (i) or (ii).
iv. A nucleotide sequence which codes for an amino acid sequence which has an identity of at least 98% with SEQ ID NO: 11.

3. The plant as claimed in claim 1 and claim 2, being **characterized by** SEQ ID NO: 10 or the chromosomal segment being an interval between the marker loci AX_99567860 and AX_99500666 on chromosome 4R from a donor selected from the ergot resistant genotypes of Gülzow germplasm.

4. The plant as claimed in claim 1 and claim 2, being **characterized by** the chromosomal segment having one or more of the following marker loci of the donor: Ergot resistant haplotype (amplification product of the primer with primer pairs of SEQ ID NO: 3, 4 and 5.

5. The plant as claimed in claim 1 and claim 2, being **characterized by** the chromosomal segment being no larger than 85.9 kb between 635894090-635980002 mbp on 4R chromosome.

6. The plant as claimed in claim 1 and claim 2, being **characterized by** the plant being an inbred plant, an edited plant and a double haploid plant with nucleotide sequences described above.

7. The plant as claimed in claim 1 and claim 2, being **characterized by** an enhanced resistance against a pathogen, preferably against a fungus *Clavicep pupurea* (Fr.).

8. Use of KASP markers with and without deletion named as SEQ ID NO: 6, 7 and 8 designed in this study for selection of material for ergot resistance. KASP marker sequences are provided in two pairs (with and without deletion) .

9. The plant as claimed in claim 1 and claim 2, being **characterized by** the plant being of the genus Secale.

10. An expression cassette, recombinant DNA or vector comprising a nucleic acid molecule as claimed in claim 2.
